(19) Europäisches Patentamt
European Patent Office
Office européen des brevets

(11) Veröffentlichungsnummer: **0 316 792 B1**

(12) **EUROPÄISCHE PATENTSCHRIFT**

(45) Veröffentlichungstag der Patentschrift: **19.01.94**

(51) Int. Cl.[5]: **C08F 283/06**, A61L 15/00, C08L 51/08

(21) Anmeldenummer: **88118802.3**

(22) Anmeldetag: **11.11.88**

# (54) Hydrophile quellbare Pfropfpolymerisate, deren Herstellung und Verwendung.

(30) Priorität: **13.11.87 DE 3738602**

(43) Veröffentlichungstag der Anmeldung:
**24.05.89 Patentblatt 89/21**

(45) Bekanntmachung des Hinweises auf die Patenterteilung:
**19.01.94 Patentblatt 94/03**

(84) Benannte Vertragsstaaten:
**BE CH DE ES FR GB GR IT LI NL SE**

(56) Entgegenhaltungen:
**EP-A- 0 006 605**
**EP-A- 0 011 833**
**DE-A- 3 401 813**
**LU-A- 56 265**

(73) Patentinhaber: **CASSELLA Aktiengesellschaft**
**Hanauer Landstrasse 526**
**D-60386 Frankturt(DE)**

(72) Erfinder: **Engelhardt, Friedrich, Dr.**
**Hünfelder Strasse 20**
**D-6000 Frankturt/Main 61(DE)**
Erfinder: **Riegel, Ullrich**
**Steinäckerstrasse 6**
**D-6000 Frankturt/Main 61(DE)**

(74) Vertreter: **Urbach, Hans-Georg, Dr. et al**
**Hanauer Landstrasse 526**
**D-60386 Frankturt (DE)**

EP 0 316 792 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft hydrophile, quellbare Pfropfpolymerisate, die in statistischer Verteilung zu 0,5 bis 20 Gew.% aus Resten der allgemeinen Formel I

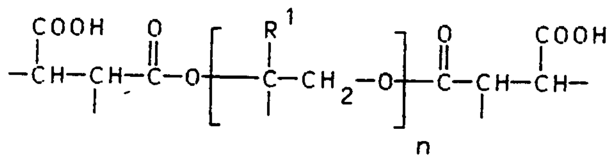

(I)

zu 79 bis 99 Gew.% aus eine saure Gruppe enthaltenden Resten der allgemeinen Formel II

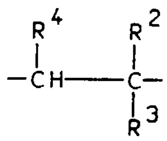

(II)

und zu 0,1 bis 2 Gew.% aus Resten eines Vernetzers, die aus Monomeren mit mindestens zwei olefinisch ungesättigten Doppelbindungen hervorgegangen sind, bestehen, wobei
n 2 bis 300,
$R^1$ Wasserstoff oder Methyl,
$R^2$ Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

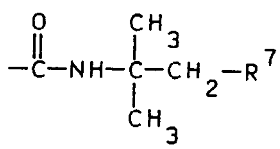

worin $R^7$ für die Sulfonylgruppe oder die Phosphonylgruppe steht,
$R^4$ Wasserstoff, Methyl, Ethyl oder die Carboxylgruppe bedeuten,
sowie ihre Herstellung und Verwendung als Absorptionsmittel für Wasser und wäßrige Lösungen, zum Beispiel in Hygieneartikeln, zur Bodenverbesserung oder als Filtrationshilfsmittel.

Quellbare Polymere, die wäßrige Lösungen absorbieren, werden für die Herstellung von Tampons, Windeln, Damenbinden und anderen Hygieneartikeln sowie als wasserzurückhaltende Mittel im landwirtschaftlichen Gartenbau verwendet.

Zu bekannten Absorptionsharzen dieses Typs gehören vernetzte Carboxymethylcellulose, teilweise vernetztes Polyalkylenoxid, Hydrolysate von Stärke-Acrylnitril-Pfropfcopolymeren oder teilweise vernetzte Polyacrylsäuresalze.

Diese bekannten Polymerisate zeigen durchweg Nachteile, insbesondere bei der Absorption wäßriger Elektrolytlösung sowie Blut und Urin.

Bei hohem Absorptionsvermögen werden nach dem derzeitigen Stand der Technik zu geringe Gelfestigkeiten der gequollenen Polymerpartikel erreicht. Es bilden sich klebrige Massen, welche die Saugfähigkeit der damit hergestellten Produkte verschlechtern.

Es ist bekannt, daß durch Erhöhung der Vernetzungsdichte die Gelfestigkeit sowie die Geschwindigkeit der Flüssigkeitsaufnahme erhöht werden kann, dadurch jedoch gleichzeitig die Absorptionskapazität herabgesetzt wird. Diese Vorgehensweise ist insofern unerwünscht als die Absorptionskapazität die wichtigste Eigenschaft des Polymeren ist.

Aufgabe der vorliegenden Erfindung ist es, modifizierte, Polymere, die wäßrige Lösungen absorbieren, bereitzustellen, welche eine hohe Absorptionsrate aufweisen und dabei im gequollenen Zustand nicht klebende Hydrogelpartikel hoher Gelfestigkeit bilden.

Überraschenderweise wurde nun gefunden, daß das gewünschte Eigenschaftsprofil durch die erfindungsgemäßen Pfropfpolymerisate erreicht wird, da deren makromolekulares Netzwerk physikalisch eine

Erhöhung der Gelfestigkeit oder Gelstärke des gequollenen Polymeren sowie eine verbesserte Elektrolyttoleranz bewirkt.

Bevorzugte erfindungsgemäße Produkte bestehen zu 0,5 bis 15 Gew.% aus Resten der allgemeinen Formel I, 84 bis 99 Gew.% aus Resten der allgemeinen Formel II und 0, 1 bis 1,8 Gew.% aus vernetzenden Strukturen, die aus Monomeren mit mindestens zwei olefinisch ungesättigten Doppelbindungen hervorgegangen sind.

Besonders bevorzugte erfindungsgemäße Produkte bestehen zu 1 bis 10,5 Gew.% aus Resten der allgemeinen Formel I, 88 bis 98,5 Gew.% aus Resten der allgemeinen Formel II und 0,3 bis 1,5 Gew.% aus vernetzenden Strukturen, die aus Monomeren mit mindestens zwei olefinisch ungesättigten Doppelbindungen hervorgegangen sind.

In den erfindungsgemäßen Pfropfcopolymerisaten können die Reste der allgemeinen Formel I alle exakt die gleiche Struktur haben, sie können sich jedoch auch hinsichtlich des Restes $R^1$ und/oder der Zahl n voneinander unterscheiden. So können sich bezüglich $R^1$ Wasserstoff und Methyl in statistischer Weise abwechseln, es können aber auch größere Polymerabschnitte aufeinander folgen, in denen $R^1$ jeweils nur Wasserstoff oder nur Methyl bedeutet.

In den Resten der allgemeinen Formel II bedeutet $R^2$ bevorzugt Wasserstoff oder Methyl. $R^3$ steht bevorzugt für die Carboxylgruppe, die Sulfonylgruppe oder die Phosphonylgruppe. Besonders bevorzugt ist die Carboxylgruppe. $R^4$ bedeutet bevorzugt Wasserstoff.

Die genannten vernetzenden Strukturen können sich von allen geeigneten Monomeren mit mindestens zwei olefinisch ungesättigten Doppelbindungen ableiten.

Geeignete Monomere sind beispielsweise Verbindungen, die mindestens zwei Alkenylgruppen, zum Beispiel Vinyl oder Allyl, oder mindestens zwei Alkenoylgruppen, zum Beispiel Acrylat oder Methacrylat, enthalten.

Bevorzugt leiten sich die vernetzenden Strukturen von Monomeren ab, die 2, 3 oder 4 ethylenisch ungesättigte Doppelbindungen enthalten.

Besonders bevorzugt leiten sich die vernetzenden Strukturen von Bisacrylamidoessigsäure, Trimethylolpropantriacrylat oder Tetraallyloxyethan ab.

Ganz besonders bevorzugte erfindungsgemäße Pfropfpolymerisate sind solche, in denen mehrere der oben genannten bevorzugten oder besonders bevorzugten Merkmale enthalten sind.

Die erfindungsgemäßen Pfropfpolymerisate können durch bekannte Polymerisationsverfahren hergestellt werden. Bevorzugt ist die Polymerisation in wäßriger Lösung nach dem Verfahren der sogenannten Gelpolymerisation. Dabei werden 15-50%ige wäßrige Lösungen der Comonomeren mit bekannten geeigneten Katalysatorsystemen ohne mechanische Durchmischung unter Ausnutzung des Trommsdorff-Norrish-Effektes (Bios Final Rep. 363.22; Makromol. Chem. 1, 169 (1947)) polymerisiert.

Die Polymerisationsreaktion kann im Temperaturbereich zwischen 0°C und 130°C, vorzugsweise zwischen 10°C und 100°C, sowohl bei Normaldruck als auch unter erhöhtem Druck durchgeführt werden. Wie üblich kann die Polymerisation auch in einer Schutzgasatmosphäre, vorzugsweise unter Stickstoff, ausgeführt werden.

Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert. Butyl-hydroperoxid, Methyl-ethyl-keton-peroxid, Cumol-hydroperoxid, Azoverbindungen wie Azo-di-iso-butyro-nitril sowie anorganische Peroxiverbindungen wie $(NH_4)_2S_2O_8$ oder $K_2S_2O_8$ oder $H_2O_2$ gegebenenfalls in Kombination mit Reduktionsmitteln wie Natriumhydrogensulfit, und Eisen-II-Sulfat oder Redoxsysteme, welche als reduzierende Komponente eine aliphatische und aromatische Sulfinsäure, wie Benzolsulfinsäure und Toluolsulfinsäure oder Derivate dieser Säuren, enthalten, wie z.B. Mannichaddukte aus Sulfinsäure, Aldehyden und Aminoverbindungen, wie sie in der deutschen Patentschrift 1301566 beschrieben sind. Pro 100 g Gesamtmonomeren werden in der Regel 0,03 bis 2 g des Polymerisationsinitiators eingesetzt.

Durch mehrstündiges Nachheizen der Polymerisatgele im Temperaturbereich von 50-130°C, vorzugsweise 70-100°C, können die Qualitätseigenschaften der Polymerisate noch verbessert werden.

Die auf diesem Wege hergestellten, in Form wäßriger Gallerten vorliegenden erfindungsgemäßen Copolymerisate können nach mechanischer Zerkleinerung mit geeigneten Apparaten durch bekannte Trocknungsverfahren in fester Form erhalten werden und zum Einsatz gelangen.

Zweckmäßigerweise werden somit erfindungsgemäße Pfropfpolymerisate erhalten, wenn 0,5 bis 20 Gew.%, vorzugsweise 0,5 bis 15, insbesondere 1 bis 10,5 Gew.%, einer Polyalkylenoxidverbindung der allgemeinen Formel Ia

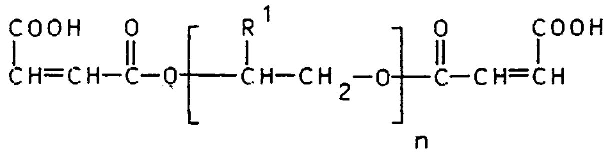 (Ia)

oder ein Alkali-, Ammonium- oder Aminsalz davon, 79 bis 99 Gew.%, vorzugsweise 84 bis 99, insbesondere 88 bis 98,5 Gew.%, einer ungesättigten Säure der allgemeinen Formel IIa

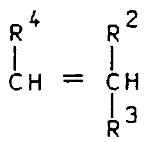 (IIa)

oder ein Alkali-, Ammonium- oder Aminsalz davon und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen, wobei die Reste $R^1$ bis $R^4$ und die Zahl n die oben genannten Bedeutungen haben, unter den Bedingungen der Gelpolymerisation umgesetzt werden.

Die Polyalkylenoxidverbindungen der allgemeinen Formel Ia können durch eine einfache Veresterungsreaktion zwischen Maleinsäureanhydrid und Polyalkylenoxiden erhalten werden. Geeignete Polyalkylenoxide sind zum Beispiel Polyethylenglykol, Polypropylenglykol, Blockcopolymere aus Polyethylenoxid und Polypropylenoxid-blöcken sowie statistische Ethylen/Propylenoxid-Copolymere.

Die Monomeren der Formel IIa sind bekannte Verbindungen, wie zum Beispiel Acrylsäure, Methacrylsäure, Vinylsulfonsäure, Maleinsäure, Fumarsäure, Crotonsäure, 2-Acrylamido-2-methyl-propansulfonsäure, 2-Acrylamido-2-methyl-propanphosphonsäure und Vinylphosphonsäure sowie deren Halbester.

Die als Vernetzer eingesetzten polyolefinischen Monomere sind gängige Produkte. Beispiele sind Bisacrylamidoessigsäure, Trimethylolpropantriacetat und Tetraallyloxyethan.

Die erfindungsgemäßen Pfropfpolymerisate eignen sich hervorragend als Absorptionsmittel für Wasser und wäßrige Lösungen, so daß sie vorteilhaft als wasserzüruckhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Die folgenden Beispiele 1 bis 13 veranschaulichen die Herstellung erfindungsgemäßer Pfropfpolymerisate.

Beispiel 1 :

In einen durch geschäumtes Kunststoffmaterial gut isolierten Polyethyleneimer mit einem Fassungsvermögen von 10 l werden 5169 g E-Wasser vorgelegt, 1000 g Natriumbicarbonat darin dispergiert und langsam 1888 g Acrylsäure so zudosiert, daß ein Überschäumen der Reaktionslösung vermieden wird, wobei sich diese auf eine Temperatur von ca. 12 - 10 °C abkühlt. Es werden nun 100 g des als Pfropfgrundlage dienenden Umsetzungsproduktes gemäß Beispiel a (siehe unten), 12 g Trimethylolpropantriacrylat und 10 g eines Natrium-Diisooctylsulfosuccinates (Rewopol V 2133 der Firma REWO, Steinau) zugegeben. Bei einer Temperatur von 10 - 12 °C werden die Initiatoren, ein Redoxsystem, bestehend aus 2,2 g 2,2'-Azobisamidinopropan-Dihydrochlorid, gelöst in 20 g Wasser, 4,4 g Kaliumperoxidisulfat, gelöst in 170 g Wasser, und 6 g Natriumpyrosulfit, gelöst in 120 g Wasser, nacheinander zugegeben und gut verrührt. Die Reaktionslösung wird daraufhin ohne Rühren stehen gelassen, wobei durch einsetzende Polymerisation, in dessen Verlauf die Temperatur bis auf ca. 85 °C ansteigt, ein festes Gel entsteht. Dieses wird anschließend mechanisch zerkleinert, bei Temperaturen über 80 °C getrocknet und gemahlen.

Das vorstehend beschriebene Produkt wurde in herkömmlicher Weise in eine Babywindel eingearbeitet und zeichnete sich hier durch eine besonders gute Flüssigkeitsretention aus.

Herstellung der Pfropfgrundlagen:

Beispiel a:

In 345 g eines Blockcopolymeren aus 1,6 mol Propylenoxid und 0,2 mol Ethylenoxid mit der OH-Zahl 65, werden unter Rühren bei Raumtemperatur 39,2 g Maleinsäureanhydrid eingetragen und diese Mischung unter Rühren auf 80 °C erhitzt. Das Maleinsäureanhydrid löst sich dabei unter schwach exothermer Reaktion auf, es entsteht eine klare schwach gelblich gefärbte Lösung.

In analoger Weise wurden folgende Umsetzungsprodukte hergestellt:

Beispiel b:

488 g eines Copolymeren aus 1,4 mol Propylenoxid und 0,45 mol Ethylenoxid mit der OH-Zahl 46 sowie 39,2 g Maleinsäureanhydrid.

Beispiel c:

311,8 g eines Copolymeren aus 1,05 mol Propylenoxid und 0,9 mol Ethylenoxid mit der OH-Zahl 36 sowie 19,6 g Maleinsäureanhydrid.

Beispiel d:

330 g eines Copolymeren aus 0,35 mol Propylenoxid und 1,82 mol Ethylenoxid mit der OH-Zahl 17 sowie 9,8 g Maleinsäureanhydrid.

Beispiel e:

300 g Polyethylenglykol mit einem Molgewicht von 300 und 196 g Maleinsäureanhydrid. Während der gesamten Reaktionszeit wird ein schwacher Stickstoffstrom eingeleitet.

Beispiel f:

300 g Polyethylenglykol mit einem Molgewicht von 1500 und 39,2 g Maleinsäureanhydrid. Während der gesamten Reaktionszeit wird ein schwacher Stickstoffstrom eingeleitet.

Beispiel g:

350 g Propylenglykol mit einem Molgewicht von 1750 und 39,2 g Maleinsäureanhydrid. Während der gesamten Reaktionszeit wird ein schwacher Stickstoffstrom eingeleitet.

Beispiel 2:

In einem 10 Liter-Kunststoffeimer werden 4419 g Eis und 1888 g Acrylsäure vorgelegt und langsam 1573 g NaOH 50%ig zudosiert, anschließend 100 g des als Pfropfgrundlage dienenden Umsetzungsproduktes gemäß Beispiel 1a, 12 g Bisacrylamidoessigsäure, dispergiert in 100 g Wasser und durch Zugabe von NaOH gelöst und auf pH 6 eingestellt, und 10 g Rewopol V 2133 zugegeben. Die Reaktionslösung wird auf 20 °C eingestellt und anschließend mit den Initiatoren, ein Redoxsystem bestehend aus 6 g Kaliumperoxidisulfat, gelöst in 170 g Wasser, und 0,15 g Ascorbinsäure, gelöst in 120 g Wasser, versetzt und ohne Rühren stehen gelassen. Das durch Polymerisation entstehende Gel wird anschließend mechanisch zerkleinert, bei Temperaturen über 80 °C getrocknet und gemahlen.

Beispiel 3:

In einem 10 Liter-Polyethyleneimer werden 5250 g E-Wasser, 1888 g Acrylsäure und 100 g des als Pfropfgrundlage dienenden Umsetzungsproduktes gemäß Beispiel 1a vorgelegt. Es werden 12 g Tetraallyloxyethan und 10 g Rewopol V 2133 eingerührt. Nach Einstellen der Reaktionslösung auf 18 - 20 °C werden die Initiatoren, 6 g Kaliumperoxidisulfat in 170 g Wasser und 0,2 g Ascorbinsäure in 20 g Wasser nacheinander zugegeben und das Reaktionsgefäß gut isoliert ohne Rühren stehen gelassen. Nach einsetzender Reaktion steigt die Temperatur bis auf ca. 90 °C an, und es entsteht ein festes Gel. Dieses wird mechanisch durch einen Extruder zerkleinert, dem kontinuierlich 1555 g NaOH 50%ig zudosiert werden, wobei teilweise Verdampfung des Wassers erfolgt. Das flockige Polymer wird anschließend bei Temperaturen über 80 °C endgetrocknet und gemahlen.

Weitere Beispiele zur Herstellung erfindungsgemäßer Pfropfpolymerisate gemäß der hier beschriebenen Beispiele 1 und 2 sind in folgender Tabelle zusammengefaßt. Die Mengenangaben bedeuten Gewichts% bezogen auf Gesamtmonomeranteil.

Folgende Abkürzungen werden benutzt:

| | |
|---|---|
| AS: | Acrylsäure |
| MAS: | Methacrylsäure |
| CTS: | Crotonsäure |
| VPS: | Vinylphosphonsäure |
| VPE: | Vinylphosphonsäurehalbester |
| AMPS: | 2-Acrylamido-2-methyl-propansulfonsäure |
| AMPP: | 2-Acrylamido-2-methyl-propanphosphonsäure |
| BAAE: | Bisacrylamidoessigsäure |
| TMPTA: | Trimethylolpropantriacetat |
| TAE: | Tetraallyloxyethan |

| Beispiel | hergestellt analog Beispiel | AS (%) | MAS (%) | AMPS (%) | AMPP (%) | VPS (%) | VPE (%) | CTS (%) | Pfropfgrundlage gemäß Beispiel | Pfropfgrundlage (%) | BAAE (%) | TMPTA (%) | TAE (%) | Neutralisationsgrad (%) |
|---|---|---|---|---|---|---|---|---|---|---|---|---|---|---|
| 4 | 1 | 94,4 | | | | | | | 1g | 5 | | 0,6 | | 45 |
| 5 | 1 | 94,4 | | | | | | | 1d | 5 | | 0,6 | | 45 |
| 6 | 1 | 89,4 | | | | | | | 1d | 10 | | 0,6 | | 45 |
| 7 | 1 | 94,4 | | | | | | | 1e | 5 | | 0,6 | | 45 |
| 8 | 1 | 94,4 | | | | | | | 1c | 5 | | 0,6 | | 45 |
| 9 | 1 | 94,4 | | | | | | | 1b | 5 | | 0,6 | | 45 |
| 10 | 1 | 88,5 | | | | | | | 1b | 10 | | 1,5 | | 70 |
| 11 | 1 | 94,4 | | | | | | | 1f | 5 | | 0,6 | | 45 |
| 12 | 2 | 89,4 | | | | | | | 1a | 10 | 0,6 | | | 75 |
| 13 | 2 | 89,4 | | | | | | | 1a | 10 | | | 0,6 | 78 |
| 14 | 1 | 98,4 | | | | | | | 1c | 1 | | 0,6 | | 45 |
| 15 | 1 | 70,0 | 10,0 | 9,5 | | | | | 1d | 10 | | | 0,5 | 48 |
| 16 | 2 | 65,0 | | 25,0 | | 4,0 | | | 1c | 5 | 1,0 | | | 45 |
| 17 | 2 | 75,0 | 5,0 | 10,0 | | | 4,2 | | 1c | 5 | | 0,8 | | 60 |
| 18 | 2 | 85,0 | | 5,0 | 4,5 | | | | 1c | 5 | 0,5 | | | 70 |
| 19 | 1 | 72,0 | | 20,0 | | 4,2 | | | 1b | 3 | 0,8 | | | 80 |
| 20 | 1 | 81,0 | 10,0 | | | | 4,0 | | 1c | 4 | | 1,0 | | 36 |
| 21 | 2 | 90,0 | | | 4,6 | | | | 1c | 5 | | | 0,4 | 25 |
| 22 | 2 | 79,0 | | 19,0 | | | | | 1f | 1 | | | 1,0 | 40 |
| 23 | 1 | 72,0 | | 19,3 | | | | 5,0 | 1c | 3 | | | 0,7 | 48 |
| 24 | 1 | 90,0 | | | | 1,0 | | | 1c | 8 | | | 1,0 | 32 |

## Patentansprüche

### Patentansprüche für folgende Vertragsstaaten : BE, DE, GB, NL, CH, GR, SE, LI, FR, IT

1. Hydrophile, quellbare Pfropfpolymerisate, die in statistischer Verteilung zu 0,5 bis 20 Gew.% aus Resten der allgemeinen Formel I

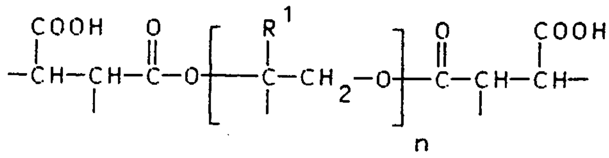

(I)

79 bis 99 Gew.% aus eine saure Gruppe enthaltenden Resten der allgemeinen Formel II

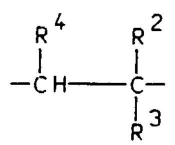

(II)

und 0,1 bis 2 Gew.% aus vernetzenden Strukturen, die aus Monomeren mit mindestens zwei olefinisch ungesättigten Doppelbindungen hervorgegangen sind, bestehen, wobei
n 2 bis 300,
$R^1$ Wasserstoff oder Methyl,
$R^2$ Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

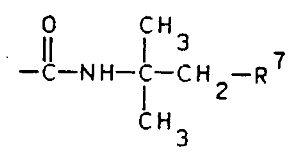

worin $R^7$ für die Sulfonylgruppe oder die Phosphonylgruppe steht,
$R^4$ Wasserstoff, Methyl, Ethyl oder die Carboxylgruppe bedeuten.

2. Pfropfpolymerisate gemäß Anspruch 1, dadurch gekennzeichnet, daß sie in statistischer Verteilung zu 1 bis 10,5 Gew.% aus Resten der allgemeinen Formel I, zu 88 bis 98,5 Gew.% aus Resten der allgemeinen Formel II und zu 0,3 bis 1,5 Gew.% aus vernetzenden Strukturen bestehen.

3. Pfropfpolymerisate gemäß Ansprüchen 1 und/oder 2, dadurch gekennzeichnet, daß sich die Reste der allgemeinen Formel I hinsichtlich des Restes $R^1$ und/oder der Zahl n voneinander unterscheiden.

4. Pfropfpolymerisate nach einem oder mehreren der Ansprüche 1 bis 3, dadurch gekennzeichnet, daß in den Resten der allgemeinen Formel II $R^2$ Wasserstoff oder Methyl, $R^3$ die Carboxylgruppe, die Sulfonylgruppe oder die Phosphonylgruppe und $R^4$ Wasserstoff bedeuten.

5. Pfropfpolymerisate nach einem oder mehreren der Ansprüche 1 bis 4, dadurch gekennzeichnet, daß in den Resten der allgemeinen Formel II $R^3$ die Carboxylgruppe bedeutet.

6. Pfropfpolymerisate nach einem oder mehreren der Ansprüche 1 bis 5, dadurch gekennzeichnet, daß sich die vernetzenden Strukturen von Monomeren mit mindestens zwei Alkenylgruppen oder mindestens zwei Alkenoylgruppen, insbesondere von Bisacrylamidoessigsäure, Trimethylolpropantriacrylat oder Tetraallyloxyethan ableiten.

**7.** Verfahren zur Herstellung der in den Ansprüchen 1 bis 6 beanspruchten Pfropfpolymerisate, dadurch gekennzeichnet, daß 0,5 bis 20 Gew.%, vorzugsweise 0,5 bis 15, insbesondere 1 bis 10,5 Gew.%, einer Polyalkylenoxidverbindung der allgemeinen Formel I a

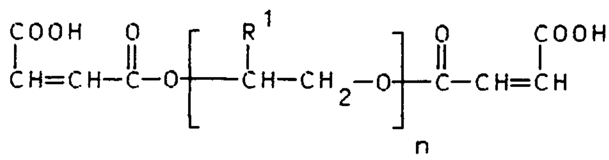

(Ia)

oder ein Alkali-, Ammonium- oder Aminsalz davon, 79 bis 99 Gew.%, vorzugsweise 84 bis 99, insbesondere 88 bis 98,5 Gew.%, einer ungesättigten Säure der allgemeinen Formel II a

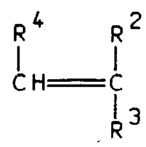

(IIa)

oder ein Alkali-, Ammonium- oder Aminsalz davon, und 0,1 bis 2 Gew.%, vorzugsweise 0,1 bis 1,8, insbesondere 0,3 bis 1,5 Gew.%, eines Monomeren mit mindestens zwei olefinisch ungesättigten Doppelbindungen unter den Bedingungen der Gelpolymerisation umgesetzt werden.

**8.** Verwendung der in den Ansprüchen 1 bis 6 beanspruchten Pfropfpolymerisate als Absorptionsmittel für Wasser und wäßrige Lösungen.

**9.** Verwendung der in den Ansprüchen 1 bis 6 beanspruchten Pfropfpolymerisate gemäß Anspruch 8, dadurch gekennzeichnet, daß die Pfropfpolymerisate in Hygieneartikeln wie Windeln, Tampons oder Damenbinden eingesetzt werden.

**Patentansprüche für folgenden Vertragsstaat : ES**

**1.** Verfahren zur Herstellung hydrophiler, quellbarer Pfropfpolymerisate, die in statistischer Verteilung zu 0,5 bis 20 Gew.% aus Resten der allgemeinen Formel I

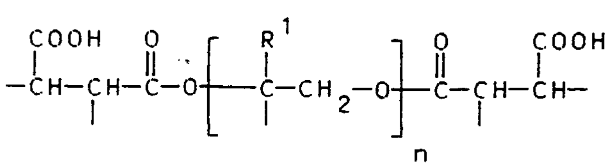

(I)

79 bis 99 Gew.% aus eine saure Gruppe enthaltenden Resten der allgemeinen Formel II

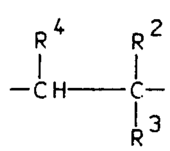

(II)

und 0,1 bis 2 Gew.% aus vernetzenden Strukturen, die aus Monomeren mit mindestens zwei olefinisch ungesättigten Doppelbindungen hervorgegangen sind, bestehen, wobei
n 2 bis 300,
$R^1$ Wasserstoff oder Methyl,
$R^2$ Wasserstoff, Methyl oder Ethyl,
$R^3$ die Carboxylgruppe, die Sulfonylgruppe, die Phosphonylgruppe, die gegebenenfalls mit Alkanol mit 1 bis 4 Kohlenstoffatomen verestert sein kann, oder eine Gruppe der Formel

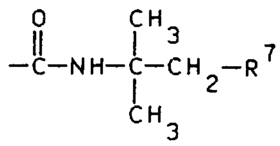

worin $R^7$ für die Sulfonylgruppe oder die Phosphonylgruppe steht, $R^4$ Wasserstoff, Methyl, Ethyl oder die Carboxylgruppe bedeuten, dadurch gekennzeichnet, daß 0,5 bis 20 Gew.% einer Polyalkylenoxidverbindung der allgemeinen Formel I a

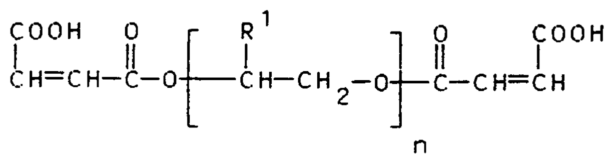

(I a)

oder ein Alkali-, Ammonium- oder Aminsalz davon, 79 bis 99 Gew. % einer ungesättigten Säure der allgemeinen Formel II a

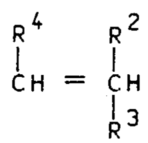

(IIa)

oder ein Alkali-, Ammonium- oder Aminsalz davon, und 0,1 bis 2 Gew. % eines Monomeren mit mindestens zwei olefinisch ungesättigten Doppelbindungen unter den Bedingungen der Gelpolymerisation umgesetzt werden.

2. Verfahren gemäß Anspruch 1, dadurch gekennzeichnet, daß die Pfropfpolymerisate in statistischer Verteilung zu 1 bis 10,5 Gew.% aus Resten der allgemeinen Formel I, zu 88 bis 98,5 Gew.% aus Resten der allgemeinen Formel II und zu 0,3 bis 1,5 Gew.% aus vernetzenden Strukturen bestehen.

3. Verfahren gemäß Ansprüchen 1 und/oder 2, dadurch gekennzeichnet, daß sich die Reste der allgemeinen Formel I hinsichtlich des Restes $R^1$ und/oder der Zahl n voneinander unterscheiden.

4. Verfahren nach einem oder mehreren der Ansprüche 1 bis 3, dadurch gekennzeichnet, daß in den Resten der allgemeinen Formel II $R^2$ Wasserstoff oder Methyl, $R^3$ die Carboxylgruppe, die Sulfonylgruppe oder die Phosphonylgruppe und $R^4$ Wasserstoff bedeuten.

5. Verfahren nach einem oder mehreren der Ansprüche 1 bis 4, dadurch gekennzeichnet, daß in den Resten der allgemeinen Formel II $R^3$ die Carboxylgruppe bedeutet.

6. Verfahren nach einem oder mehreren der Ansprüche 1 bis 5, dadurch gekennzeichnet, daß sich die vernetzenden Strukturen von Monomeren mit mindestens zwei Alkenylgruppen oder mindestens zwei Alkenoylgruppen, insbesondere von Bisacrylamidoessigsäure, Trimethylolpropantriacrylat oder Tetraallyloxyethan ableiten.

7. Verwendung der nach den Ansprüchen 1 bis 6 hergestellten Pfropfpolymerisate als Absorptionsmittel für Wasser und wäßrige Lösungen.

8. Verwendung der nach den Ansprüchen 1 bis 6 hergestellten Pfropfpolymerisate gemäß Anspruch 7, dadurch gekennzeichnet, daß die Pfropfpolymerisate in Hygieneartikeln wie Windeln, Tampons oder Damenbinden eingesetzt werden.

## Claims

**Claims for the following Contracting States : BE, DE, GB, NL, CH, GR SE, LI, FR, IT**

**1.** Hydrophilic swellable graft polymers which consist, in random distribution, to the extent of 0.5 to 20 % by weight of radicals of the general formula I

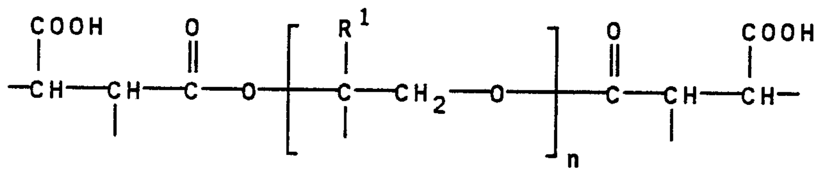

(I)

to the extent of 79 to 99 % by weight of radicals containing an acid group, of the general formula II

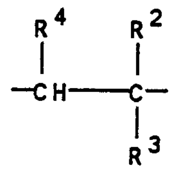

(II)

and to the extent of 0.1 to 2 % by weight of crosslinking structures originating from monomers with at least two olefinically unsaturated double bonds, wherein
n denotes 2 to 300
$R^1$ denotes hydrogen or methyl,
$R^2$ denotes hydrogen, methyl or ethyl,
$R^3$ denotes the carboxyl group, the sulphonyl group, the phosphonyl group, which can optionally be esterified with alkanol with 1 to 4 carbon atoms, or a group of the formula

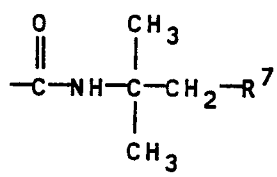

wherein $R^7$ stands for the sulphonyl group or the phosphonyl group, and
$R^4$ denotes hydrogen, methyl, ethyl or the carboxyl group.

**2.** Graft polymers according to Claim 1, characterized in that they consist, in random distribution, to the extent of 1 to 10.5 % by weight of radicals of the general formula I, to the extent of 88 to 98.5 % by weight of radicals of the general formula II and to the extent of 0.3 to 1.5 % by weight of crosslinking structures.

**3.** Graft polymers according to Claims 1 and /or 2, characterized in that the radicals of the general formula I differ from one another in respect of the radical $R^1$ and/or the number n.

**4.** Graft polymers according to one or more of Claims 1 to 3, characterized in that in the radicals of the general formula II, $R^2$ denotes hydrogen or methyl, $R^3$ denotes the carboxyl group, the sulphonyl group or the phosphonyl group and $R^4$ denotes hydrogen.

**5.** Graft polymers according to one or more of Claims 1 to 4, characterized in that in the radicals of the general formula II, $R^3$ denotes the carboxyl group.

**6.** Graft polymers according to one or more of Claims 1 to 5, characterized in that the crosslinking structures are derived from monomers with at least two alkenyl groups or at least two alkenoyl groups,

in particular from bisacrylamidoacetic acid, trimethylolpropane triacrylate or tetraallyloxyethane.

**7.** Process for the preparation of the graft polymers claimed in Claims 1 to 6, characterized in that 0.5 to 20 % by weight, preferably 0.5 to 15 and in particular 1 to 10.5 % by weight, of a polyalkylene oxide compound of the general formula Ia

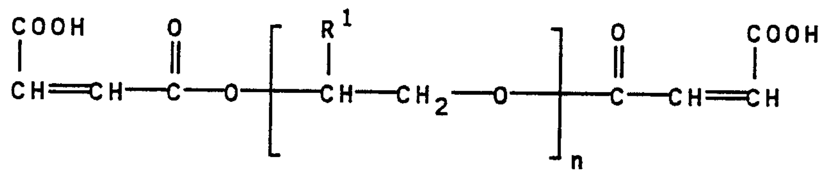

(Ia)

or an alkali metal salt, ammonium salt or amine salt thereof, 79 to 99 % by weight, preferably 84 to 99 and in particular 88 to 98.5 % by weight, of an unsaturated acid of the general formula IIa

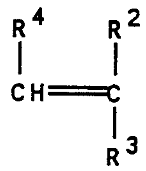

(IIa)

or an alkali metal salt, ammonium salt or amine salt thereof, and 0.1 to 2 % by weight, preferably 0.1 to 1.8 and in particular 0.3 to 1.5 % by weight, of a monomer with at least two olefinically unsaturated double bonds are reacted under the conditions of gel polymerization.

**8.** Use of the graft polymers claimed in Claims 1 to 6 as absorbants for water and aqueous solutions.

**9.** Use of the graft polymers claimed in Claims 1 to 6 according to Claim 8, characterized in that the graft polymers are used in hygiene articles, such as nappies, tampons or sanitary towels.

**Claims for the following Contracting State : ES**

**1.** Process for preparing hydrophilic swellable graft polymers which consist, in random distribution, to the extent of 0.5 to 20 % by weight of radicals of the general formula I

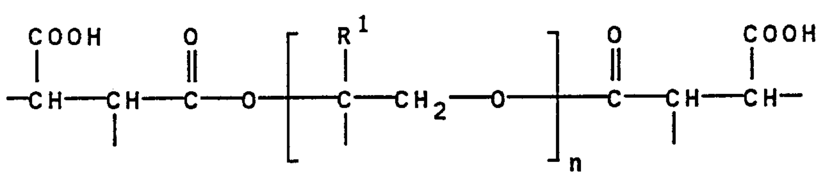

(I)

to the extent of 79 to 99 % by weight of radicals containing an acid group, of the general formula II

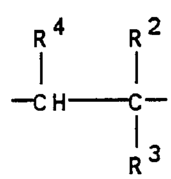

(II)

and to the extent of 0.1 to 2 % by weight of crosslinking structures originating from monomers with at least two olefinically unsaturated double bonds, wherein
n denotes 2 to 300
$R^1$ denotes hydrogen or methyl,

$R^2$ denotes hydrogen, methyl or ethyl,
$R^3$ denotes the carboxyl group, the sulphonyl group, the phosphonyl group, which can optionally be esterified with alkanol with 1 to 4 carbon atoms, or a group of the formula

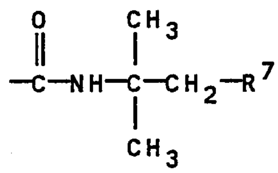

wherein $R^7$ stands for the sulphonyl group or the phosphonyl group, and
$R^4$ denotes hydrogen, methyl, ethyl or the carboxyl group, characterized in that 0.5 to 20 % by weight of a polyalkylene oxide compound of the general formula Ia

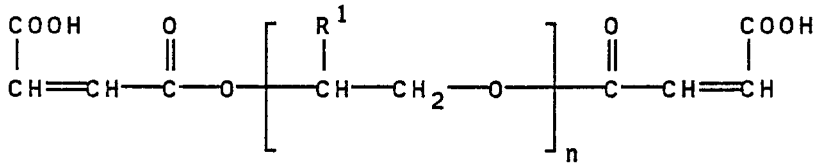 (Ia)

or an alkali metal salt, ammonium salt or amine salt thereof, 79 to 99 % by weight of an unsaturated acid of the general formula IIa

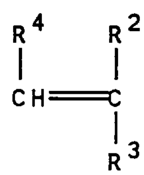 (IIa)

or an alkali metal salt, ammonium salt or amine salt thereof, and 0.1 to 2 % by weight of a monomer with at least two olefinically unsaturated double bonds are reacted under the conditions of gel polymerization.

**2.** Process according to Claim 1, characterized in that the graft polymers consist, in random distribution, to the extent of 1 to 10.5 % by weight of radicals of the general formula I, to the extent of 88 to 98.5 % by weight of radicals of the general formula II and to the extent of 0.3 to 1.5 % by weight of crosslinking structures.

**3.** Process according to Claims 1 and /or 2, characterized in that the radicals of the general formula I differ from one another in respect of the radical $R^1$ and/or the number n.

**4.** Process according to one or more of Claims 1 to 3, characterized in that in the radicals of the general formula II, $R^2$ denotes hydrogen or methyl, $R^3$ denotes the carboxyl group, the sulphonyl group or the phosphonyl group and $R^4$ denotes hydrogen.

**5.** Process according to one or more of Claims 1 to 4, characterized in that in the radicals of the general formula II, $R^3$ denotes the carboxyl group.

**6.** Process according to one or more of Claims 1 to 5, characterized in that the crosslinking structures are derived from monomers with at least two alkenyl groups or at least two alkenoyl groups, in particular from bisacrylamidoacetic acid, trimethylolpropane triacrylate or tetraallyloxyethane.

7. Use of the graft polymers prepared according to Claims 1 to 6 as absorbants for water and aqueous solutions.

8. Use of the graft polymers prepared according to Claims 1 to 6, according to Claim 7, characterized in that the graft polymers are used in hygiene articles, such as nappies, tampons or sanitary towels.

**Revendications**

**Revendications pour les Etats contractants suivants : BE, DE, GB, NL, CH, GR, SE, LI, FR, IT**

1. Polymères de greffage, gonflables et hydrophiles qui, en répartition statistique, consistent pour 0,5 à 20 % en poids en des restes de formule générale I

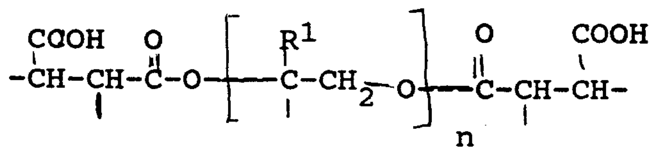

(I)

en 79 à 99 % en poids de restes de formule générale II

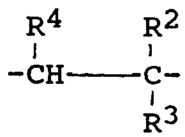

(II)

contenant un groupe acide, et en 0,1 à 2 % en poids de structures réticulantes, provenant de monomères comportant au moins deux doubles liaisons d'insaturation oléfinique, formules dans lesquelles n vaut 2 à 300,

$R^1$ représente un atome d'hydrogène ou un groupe méthyle,

$R^2$ représente un atome d'hydrogène ou un groupe méthyle ou éthyle,

$R^3$ représente le groupe carboxyle,le groupe sulfonyle, le groupe phosphonyle, qui peut éventuellement être éthérifié par un alcanol ayant 1 à 4 atomes de carbone, ou un groupe de formule

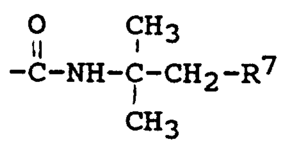

dans laquelle $R^7$ représente le groupe sulfonyle ou le groupe phosphonyle,
et $R^4$ représente un atome d'hydrogène, ou un groupe méthyle, éthyle, ou carboxyle

2. Polymères greffés selon la revendication 1, caractérisés en ce qu'ils consistent, en répartition statistique, pour 1 à 10,5 % en poids en des restes de formule générale I, pour 88 à 98,5 % en poids en des restes de formule générale II et pour 0,3 à 1,5 % en poids en des structures réticulantes.

3. Polymères greffés selon les revendications 1 et/ou 2, caractérisés en ce que les restes de formule générale I diffèrent l'un de l'autre par le sens du reste $R^1$ et/ou par la valeur du nombre n.

4. Polymères greffés selon une ou plusieurs des revendications 1 à 3, caractérisés en ce que, dans les restes de formule générale II, $R^2$ représente un atome d'hydrogène ou un groupe méthyle, $R^3$ représente le groupe carboxyle, le groupe sulfonyle ou le groupe phosphonyle, et $R^4$ représente un atome d'hydrogène

**5.** Polymères greffés selon une ou plusieurs des revendications 1 à 4, caractérisés en ce que, dans les restes de formule générale II, $R^3$ représente le groupe carboxyle.

**6.** Polymères greffés selon une ou plusieurs des revendications 1 à 5, caractérisés en ce que les structures réticulantes dérivent de monomères comportant au moins deux groupes alcényles ou au moins deux groupes alcénoyles, en particulier ces structures dérivent de l'acide bisacrylamidoacétique, du triacrylate de triméthylolpropane ou du tétrallyloxyéthane.

**7.** Procédé pour préparer les polymères greffés revendiqués dans les revendications 1 à 6, procédé caractérisé en ce qu'on fait réagir, dans des conditions de polymérisation avec formation d'un gel, 0,5 à 20 % en poids, avantageusement 0,5 à 15, notamment 1 à 10,5 % en poids d'un poly(oxyde d'alkylène) de formule générale Ia:

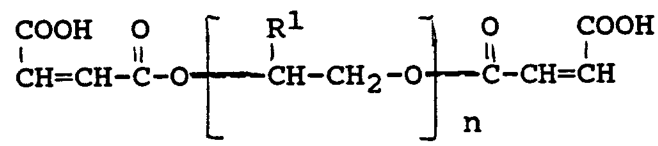

(Ia)

ou un sel alcalin, d'ammonium ou d'amine dérivant de ce composé, 79 à 99 % en poids, avantageusement 84 à 99, en particulier 88 à 98,5 % en poids, d'un acide insaturé répondant à la formule générale IIa :

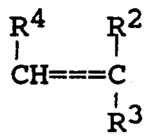

(IIa)

ou d'un sel alcalin, d'ammonium ou d'amine dérivant de cet acide, et 0,1 à 2% en poids, avantageusement 0,1 à 1,8 , notamment 0,3 à 1,5 % en poids d'un monomère comportant au moins deux doubles liaisons d'insaturation oléfinique .

**8.** Utilisation des polymères revendiqués dans les revendications 1 à 6 comme agent d'absorption de l'eau et de solutions aqueuses.

**9.** Utilisation des polymères greffés revendiqués dans les revendications 1 à 6, selon la revendication 8, caractérisée en ce qu'on utilise les polymères greffés dans des articles d'hygiène comme des langes ou couches, des tampons et des serviettes hygiéniques ou périodiques.

**Revendications pour l'Etat contractant suivant : ES**

**1.** Procédé de préparation de polymères greffés gonflables et hydrophiles qui, en répartition statistique, consistent pour 0,5 à 20 % en poids en des restes de formule générale I

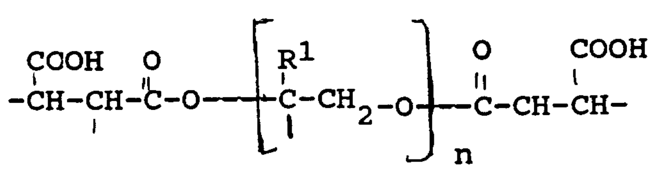

(I),

en 79 à 99 % en poids de restes de formule générale II

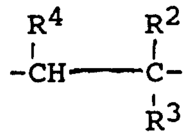

(II)

contenant un groupe acide, et en 0,1 à 2 % en poids de structures réticulantes, provenant de monomères comportant au moins deux doubles liaisons d'insaturation oléfinique, [formules dans lesquelles n vaut 2 à 300,

$R^1$ représente un atome d'hydrogène ou un groupe méthyle,

$R^2$ représente un atome d'hydrogène ou un groupe méthyle ou éthyle,

$R^3$ représente le groupe carboxyle, le groupe sulfonyle, le groupe phosphonyle, qui peut éventuellement être éthérifié par un alcanol ayant 1 à 4 atomes de carbone, ou un groupe de formule

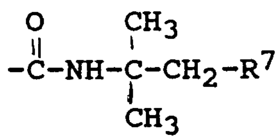

dans laquelle $R^7$ représente le groupe sulfonyle ou le groupe phosphonyle,

et $R^4$ représente un atome d'hydrogène, ou un groupe méthyle, éthyle, ou carboxyle], procédé caractérisé en ce qu'on fait réagir, dans des conditions de polymérisation avec formation d'un gel, 0,5 à 20 % en poids, avantageusement 0,5 à 15, notamment 1 à 10,5 % en poids d'un poly(oxyde d'alkylène) de formule générale Ia:

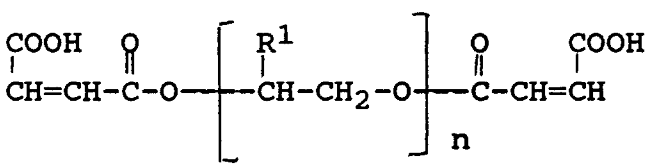

(Ia)

ou un sel alcalin, d'ammonium ou d'amine dérivant de ce composé, 79 à 99 % en poids, avantageusement 84 à 99, en particulier 88 à 98,5 % en poids, d'un acide insaturé répondant à la formule générale IIa :

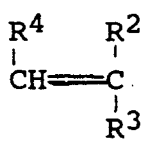

(IIa)

ou d'un sel alcalin, d'ammonium ou d'amine dérivant de cet acide, et 0,1 à 2% en poids, d'un monomère comportant au moins deux doubles liaisons d'insaturation oléfinique .

2. Procédé selon la revendication 1, caractérisé en ce que les polymères greffés consistent, en répartition statistique, pour 1 à 10,5 % en poids en des restes de formule générale I, pour 88 à 98,5 % en poids en des restes de formule générale I et pour 0,3 1,5 % en poids en des structures réticulantes.

3. Procédé selon les revendications 1 et/ou 2, caractérisé en ce que les restes de formule générale I diffèrent les uns des autres par le sens du reste $R^1$ et/ou par la valeur du nombre n.

4. Procédé selon une ou plusieurs des revendications 1 à 3, caractérisé en ce que, dans les restes de formule générale II, $R^2$ représente un atome d'hydrogène ou un groupe méthyle, $R^3$ représente le groupe carboxyle, le groupe sulfonyle ou le groupe phosphonyle et $R^4$ est un atome d'hydrogène.

**5.** Procédé selon une ou plusieurs des revendications 1 à 4, caractérisé en ce que, dans les restes de formule générale II, $R^3$ représente le groupe carboxyle.

**6.** Procédé selon une ou plusieurs des revendications 1 à 5, caractérisé en ce que les structures réticulantes dérivent de monomères comportant au moins deux groupes alcényles ou au moins deux groupes alcénoyles, en particulier de l'acide bisacrylamidoacétique, du triacrylate de triméthylol propane ou du tétrallyloxyéthane.

**7.** Utilisation des polymères greffés, produits selon les revendications 1 à 6, à titre d'agent d'absorption de l'eau et de solutions aqueuses.

**8.** Utilisation des polymères greffés, produits selon les revendications 1 à 6, selon la revendication 7, utilisation caractérisée en ce qu'on utilise les polymères greffés dans des articles d'hygiène comme des langes ou couches, des tampons ou des serviettes hygiéniques ou périodiques.